# EUROPEAN PATENT SPECIFICATION

(11) **EP 3 492 086 B1**
(45) Date of publication and mention of the grant of the patent: **25.08.2021**
(21) Application number: 18000264.4
(22) Date of filing: 16.03.2018
(51) Int. Cl.: A61K 31/7068, A61K 31/203, A61P 17/02, A61P 41/00, A61P 43/00

(54) **PHARMACEUTICAL COMPOSITION COMPRISING ZEBULARINE (1-(BETA-D-RIBOFURANOSYL)-2(1H)-PYRIMIDINONE) FOR USE IN PROMOTING REGENERATION AND WOUND HEALING**
PHARMAZEUTISCHE ZUSAMMENSETZUNG ENTHALTEND ZEBULARIN (1-(BETA-D-RIBOFURANOSYL)-2(1H)-PYRIMIDINON) ZUR FÖRDERUNG DER REGENERATION UND WUNDHEILUNG
COMPOSITION PHARMACEUTIQUE COMPRENANT DE LA ZEBULARINE (1-(BETA-D-RIBOFURANOSYL)-2(1H)-PYRIMIDINONE) POUR FAVORISER LA RÉGÉNÉRATION ET LA CICATRISATION DES PLAIES

(30) Priority: 30.11.2017 PL 42367217
(43) Date of publication of application: 05.06.2019
(73) Proprietor: Uniwersytet Gdanski, 80-309 Gdansk (PL); Politechnika Gdanska, 80-233 Gdansk (PL); Gdanski Uniwersytet Medyczny, 80-210 Gdansk (PL); Instytut Biologii Doswiadczalnej im. Marcelego Nenckiego Polskiej Akademii Nauk, 02-093 Warszawa (PL); MEDVentures sp. z. o. o., 60-141 Poznan (PL); PRO SCIENCE Polska sp. z o.o., 80-302 Gdansk (PL)
(72) Inventor: Sachadyn, Pawel, 81-646 Gdynia (PL); Sosnowski, Pawel, 19-200 Ruda (PL); Sass, Piotr, 81-577 Gdynia (PL); Podolak-Popinigis, Justyna, 80-180 Borkowo (PL); Gornikiewicz, Bartosz, 85-432 Bydgoszcz (PL); Czypryn, Artur, 02-747 Warszawa (PL); Janus, Lukasz, 60-694 Poznan (PL); Mucha, Piotr, 83-110 Tczew (PL); Pikula, Michal, 80-170 Gdansk (PL); Piotrowski, Arkadiusz, 83-031 Rózyny (PL); Skowron, Piotr, 80-303 Gdansk (PL); Rodziewicz-Motowidlo, Sylwia, 83-010 Rotmanka (PL)
(74) Representative: Pawlowska, Justyna

(56) References cited:
- WO-A1-2015/139013
- WO-A2-03/012051
- WO-A2-2011/132085
- US-A1- 2007 042 976
- AURÉLIE COMPTOUR ET AL: "Lysyl oxidase-like 4 involvement in retinoic acid epithelial wound healing", SCIENTIFIC REPORTS, vol. 6, no. 1, 6 September 2016 (2016-09-06), XP055491656, DOI: 10.1038/srep32688
- JURATE SAVICKIENE ET AL: "Epigenetic changes by zebularine leading to enhanced differentiation of human promyelocytic leukemia NB4 and KG1 cells", MOLECULAR AND CELLULAR BIOCHEMISTRY, KLUWER ACADEMIC PUBLISHERS, BO, vol. 359, no. 1 - 2, 13 August 2011 (2011-08-13), pages 245-261, XP019978928, ISSN: 1573-4919, DOI: 10.1007/S11010-011-1019-7
- HU ZHIPING ET AL: "The Emerging Role of Epigenetics in Cerebral Ischemia", MOLECULAR NEUROBIOLOGY, HUMANA PRESS, US, vol. 54, no. 3, 19 February 2016 (2016-02-19), pages 1887-1905, XP036189576, ISSN: 0893-7648, DOI: 10.1007/S12035-016-9788-3 [retrieved on 2016-02-19]
- NADIA NAEEM ET AL: "DNA Methylation Inhibitors, 5-azacytidine and Zebularine Potentiate the Transdifferentiation of Rat Bone Marrow Mesenchymal Stem Cells into Cardiomyocytes", CARDIOVASCULAR THERAPEUTICS, vol. 31, no. 4, 18 July 2013 (2013-07-18), pages 201-209, XP055491305, ISSN: 1755-5914, DOI: 10.1111/j.1755-5922.2012.00320.x
- PIOTR SASS ET AL: "Epigenetic inhibitor zebularine activates ear pinna wound closure in the mouse", EBIOMEDICINE, vol. 46, 1 August 2019 (2019-08-01), pages 317-329, XP055660549, ISSN: 2352-3964, DOI: 10.1016/j.ebiom.2019.07.010

## Description

### FIELD OF INVENTION

The present invention relates to a pharmaceutical composition comprising zebularine (1-(β-D-ribofuranosyl)-2(1H)-pyrimidinone), or a pharmaceutically acceptable salt thereof, and retinoic acid, or a pharmaceutically acceptable salt thereof, for use in the promotion of regeneration and healing of wounds resulting from mechanical injuries, chemical injuries, burns, radiation injuries, surgical operations or pathological conditions. The invention furthermore relates to said composition for use as a medicament for promoting tissue regeneration and healing of wounds resulting from mechanical injuries, chemical injuries, burns, radiation injuries, surgical operations or pathological conditions.

### BACKGROUND OF THE INVENTION

### Regeneration limits in mammals

Regeneration is the ability to restore lost or damaged tissues and organs, which involves the restoration of original function and structure. In scarring, which is another response to wounding, fibrous connective tissue replaces the damaged structures. Regenerative capability in mammals is known to decrease with development. Adult mammalians exhibit physiological regeneration such as that of liver, epidermis, intestine epithelium but are regarded as incapable of epimorphic regeneration.

A decrease of regenerative and wound healing abilities manifested as non-healing and chronic wounds (Frykberg and Banks 2015) could be a side-effect of radiotherapy (Gieringer et al. 2011), chemotherapy (Falcone and Nappi 1984), as well as other conditions, in particular cancer (McCaw 1989, Rybinski et al. 2014), diabetes, and ischemia (Brem et al. 2003, Blakytny and Jude 2006).

Scarless skin wound healing observed in foetuses (Podolak-Popinigis et al. 2016) in different mammalian species, perfect heart repair following partial resection in reported murine neonates (Porrello et al. 2011) and functional regeneration of spinal cord after complete transection found in opossum pups (Fry et al. 2003) are the spectacular phenomena which imply that mammals have huge regenerative potential repressed in adulthood.

### Ear pinnae-injury model

Making 2 mm holes in the ear pinnae is a method for permanent marking of laboratory mice. As the result of healing, the holes made in the centre of ear pinnae decrease in size but remain for life. In contrast to the majority of murine strains, the MRL/MpJ mouse has been observed to close ear holes within 30 days after injury (Clark et al. 1998). The closure involves the restoration of complex tissue architecture including not only skin, but also blood vessels, muscles, cartilage, and peripheral nerves (Buckley et al. 2011) and it seems related to enhanced regenerative responses in other tissues including heart (Leferovich et al. 2001), digit stumps after amputation (Chadwick et al. 2007), retina (Xia et al. 2011), cornea (Ueno et al. 2005), spinal cord (Thuret et al. 2012), tendons (Lalley et al. 2015), articular joints (Fitzgerald et al. 2008). Therefore, the phenomenon of ear pinnae hole closure could be regarded as an indicator of regenerative capability in the whole organism. In laboratory mice, which show no ability to close ear holes spontaneously, ear pinnae regeneration could be induced pharmacologically. Ear hole closure after topical delivery of an immunophilin ligand GM284 was described in a patent pending submitted by D.E. Weinstein (US2009/0093528A1 2009). Later studies reported partial ear hole closure induction using Wnt pathway inhibitor XAV-939 (Bastakoty et al. 2015) and a prolyl hydroxylase inhibitor 1,4-DPCA (4-oxo-1H-1,10-phenanthroline-3-carboxylic acid) (Zhang et al. 2015).

### Epigenetic basis of regeneration

Organism and organ development depends on epigenetic regulation. More than that, a number of epigenetic characteristics determine the pluripotency induction in somatic cells. Epigenetic basis of regenerative potential (Gornikiewicz et al. 2013, Gornikiewicz et al. 2016, Podolak-Popinigis et al. 2016) and the involvement of epigenetic mechanisms in regeneration processes have been reported in several studies (Yakushiji et al. 2007, Powell et al. 2013, Sim et al. 2015). However, the attempts to use epigenetic agents in order to stimulate regeneration processes met with limited success (Wang et al. 2010, Tan et al. 2015).

### DNA methyltransferase inhibitors

DNA methylation is a fundamental epigenetic mechanism of gene expression regulation and it plays important roles in a number of biological processes such as development, organogenesis, aging, differentiation and de-differentiation. Impaired DNA methylation first found in neoplasms, later, was reported in a number of other malignancies. As a principle, DNA methylation of promoter regions results in repressed gene expression. The inhibition of DNA methyltransferase leads to passive demethylation during successive rounds of DNA replication, thus allowing the activation of genes silenced by DNA methylation. As DNA methylation-mediated gene repression is found in different pathologies, DNA methyltransferases inhibitors are investigated as drug candidates in different cancers, in addition to immunological disorders and neurodegenerative diseases.

The inhibitors of DNA methyltransferases could be divided into the nucleoside and non-nucleoside ones. The first are the analogues of natural nucleosides and they are incorporated into DNA during replication. Binding of the modified nucleosides incorporated into DNA leads to irreversible inactivation of DNA methyltransferases. The non-nucleoside inhibitors of DNA methyltransferase exert their action without incorporation into DNA, so they could block DNA methyltransferases in non-dividing cells. Once incorporated into DNA, the nucleoside inhibitors are possible to retain in cells longer than the non-nucleoside ones.

Two inhibitors of DNA-methyltransferase, 5-aza-2'-deoxycytidine and 5-azacytidine are used in anticancer therapies to treat myelodysplastic syndromes and acute myeloid leukaemia. The anticancer activity is explained by cytotoxic and demethylating effects, and the latter one is thought to activate cell differentiation.

### Zebularine - uses and mechanism of action

Zebularine is a cytidine analog lacking the amino group at C-4. As a nucleoside, zebularine has to be incorporated into DNA to act as a DNA methyltransferase inhibitor. This incorporation is preceded by metabolic activation: phosphorylation to zebularine monophosphate by uridine-cytidine kinase and further phosphorylation to diphosphate by nucleoside-phosphate kinase followed by the reduction to deoxyzebularine diphosphate by ribonucleotide reductase and subsequent phosphorylation to triphosphate by nucleoside-diphosphate kinase (Ben-Kasus et al. 2005). Deoxyzebularine triphosphate is utilized during DNA synthesis as a substrate of DNA polymerase. Zebularine incorporated mainly in the place of cytidine mispairs with adenine (Lee et al. 2004). Zebularine incorporated into DNA forms a stable covalent adduct with DNA methyltransferase (but unlike 5-aza-2'-deoxycytidine it is not methylated in the course of inhibition). This leads to depletion of DNA methyltransferases and DNA hypomethylation (Cheng et al. 2004). Intracellular zebularine uptake is dependent on active transport that is counter-acted by efflux through protein transporters (Arimany-Nardi et al. 2014). Oxidation to uridine by liver aldehyde oxidase in the cytosol and RNA incorporation challenge zebularine conversion to deoxyzebularine triphosphate, the substrate for DNA incorporation. In addition, the RNA incorporation is estimated to be 7-fold higher than that to DNA (Ben-Kasus et al. 2005). Further, zebularine phosphorylation can be inhibited competitively by cytidine (Ben-Kasus et al. 2005).

In contrast to two known epigenetic drugs, 5-azacytidine and 5-aza-2'-deoxycytidine, zebularine displays high stability in water with a half-life of 508 h at 37°C, at pH 7.4 (Cheng et al. 2003) and very low toxicity as observed in cell culture (Ben-Kasus et al. 2005) and animal models. No toxic effects were found in mice after treatment with 400 mg/kg body weight of intraperitoneally delivered zebularine for 78 consecutive days (Herranz et al. 2006).

The impediments in metabolic activation are probably responsible for the requirement of high zebularine doses (several hundred mg/kg body weight), but, on the other hand, its low toxicity. Low zebularine toxicity may be also explained by the fact that zebularine metabolites are endogenous substances (Beumer et al. 2006). In addition to unusually excessive doses, the use of zebularine could be complicated by its inhibitory action on thymidylate synthase and cytidine deaminase (Yoo et al. 2008). Because of these drawbacks, zebularine, though showing interesting properties, has never entered either medical use or clinical trials. Nevertheless, zebularine was tested for potential medical applications, primarily, as an anticancer drug in cell culture and animal models (Herranz et al. 2006, Tan et al. 2013, Yang et al. 2013). Zebularine was reported to show antineoplastic activity in different cancers either used alone or as a chemosensitizer (Andrade et al. 2014). Certain studies indicate other possible medical uses of zebularine. Zebularine was found to exert an anti-immunogenic effect, probably mediated through indolamine-2,3-deoxygenase induction in spleen (Liu et al. 2007), which could be used to treat autoimmune diseases or prevent immune rejection of transplants (Nittby et al. 2013). Local zebularine delivery directly before brain injury was demonstrated to exert neuroprotective action in a stroke model in the rat (Dock et al. 2015). Zebularine was also shown to potentiate transdifferentiation of mesenchymal stem cells into cardiomyocytes (Naeem et al. 2013).

### Zebularine as a potential pro-regenerative agent

DNA demethylation leads to re-activation of silenced genes, including those which induce cell pluripotency and may lead to cell transdifferentiation. Zebularine is listed among other DNA methyltransferase inhibitors in a number of patent applications as one of demethylating agents for pluripotency induction (US20110076678A1 2011) or cell transdifferentiation (US20110206644A1 2013). A Regarding the mechanism of pro-regenerative action, it should be considered that zebularine was found to have not only demethylating but also neuroprotective, cytotoxic to selected cell subsets and immunomodulatory effects.

US 2007/042976 describes the use of two other nucleoside demethylating agents, 5-aza-cititidine and 5-aza-2'deoxycytidine, to reduce collagen production in in vitro cultured human fibroblast and in human skin fragments in vitro. Collagen fibres maintain skin structure; therefore, collagen production and deposition are essential elements of physiological wound healing. An excess of collagen deposition may lead to increased fibrogenesis and scar formation, but the cited document showed only a decrease in collagen production but no evidence of resultant improvement in wound healing or reduced scar formation. However, zebularine displays different properties than 5-azacitidine and zebularine. Unlike 5-azadeoxycytidine and 5-azacytidine, zebularine shows lower activity and toxicity to cultured cells and animals (Ben-Kasus et al. 2005, Herranz et al. 2006), it is metabolized exclusively to endogenous compounds (Beumer et al. 2006), and it has a different mechanism of DNA methyltransferase inactivation (Zhou e al. 2002).

WO 2015/139013 discloses the use of another DNA methyltransferase inhibitor, 5-azacytidine (designated as Aza-CR), in scaffolds to induce tissue repair in a few in vivo models. However, it does not demonstrate any experiments with zebularine. The use of zebularine in the place of 5-azacytydine is not obvious in this context because unlike 5-azacytidine, zebularine shows low toxicity to cultured cells and animals (Ben-Kasus et al. 2005, Herranz et al. 2006), it is metabolized exclusively to endogenous compounds (Beumer et al. 2006), and it has a different mechanism of DNA methyltransferase inactivation (Zhou e al. 2002). Finally, the invention shown in WO 2015/139013 relates to the combined use of scaffolds and demethylating agents. The subject of our invention is the use of zebularine compositions without scaffolds to stimulate the regeneration of complex tissue.
Zebularine is a nucleoside inhibitor of DNA methyltransferases as well as cytidine deaminase and thymidylate synthase known under several names including: 1-(β-D-ribofuranosyl)-2(1H)-pyrimidinone, pyrimidin-2-one β-D-ribofuranoside, 4-deoxyuridine, 1- (β-D-Ribofuranosyl) -1,2-dihydropyrimidin-2-one, 2-Pyrimidone-1-β-D-riboside. Though it has been considered in cell-based therapies, zebularine has not been reported to be effectively applied to induce regeneration in an animal organism to date.

### SUMMARY OF THE INVENTION

The object of the present invention is an example of successful epigenetic regenerative therapy observed in a mammalian organism under treatment by using a composition comprising zebularine and retinoic acid.

In one aspect, a novel medical use of composition comprising zebularine and retinoic acid for promoting wound healing and complex tissue regeneration is provided.

In another aspect, the use of compositions comprising zebularine and retinoic acid for promoting wound healing and complex tissue regeneration is provided.

The invention is defined by the claims. Any subject-matter falling outside the scope of the claims is provided for information purposes only. Any references in the description to methods of treatment refer to the pharmaceutical composition and medicament according to the present invention for use in a method for treatment of the human or animal body by therapy.

### DETAILED DESCRIPTION OF THE INVENTION

The present invention relates to a pharmaceutical composition comprising zebularine, or a pharmaceutically acceptable salt thereof, and retinoic acid, or a pharmaceutically acceptable salt thereof, and at least one pharmaceutically acceptable carrier for use in the promotion of tissue regeneration and healing of wounds resulting from mechanical injuries, chemical injuries, burns, radiation injuries, surgical operations or pathological conditions.

The present invention also relates to a pharmaceutical composition comprising zebularine, or a pharmaceutically acceptable salt thereof, and retinoic acid, or a pharmaceutically acceptable salt thereof, and at least one pharmaceutically acceptable carrier for use as a medicament to promote tissue regeneration and healing of wounds resulting from mechanical injuries, chemical injuries, burns, radiation injuries, surgical operations or pathological conditions.

The present invention also relates to the composition comprising zebularine, or a pharmaceutically acceptable salt thereof, and retinoic acid, or a pharmaceutically acceptable salt thereof, and at least one pharmaceutically acceptable carrier for use as a medicament to promote tissue regeneration and healing of wounds resulting from mechanical injuries, chemical injuries, burns, radiation injuries, surgical operations or pathological conditions wherein the zebularine displays DNA demethylating activity.

The present invention also relates to the composition comprising zebularine, or a pharmaceutically acceptable salt thereof, and retinoic acid, or a pharmaceutically acceptable salt thereof, and at least one pharmaceutically acceptable carrier for use as a medicament to promote tissue regeneration and healing of wounds resulting from mechanical injuries, chemical injuries, burns, radiation injuries, surgical operations or pathological conditions wherein the zebularine activates silenced genes, preferably pluripotency genes.

The present invention also relates to the composition comprising zebularine, or a pharmaceutically acceptable salt thereof, and retinoic acid, or a pharmaceutically acceptable salt thereof, and at least one pharmaceutically acceptable carrier for use in the promotion or as a medicament to promote tissue regeneration and healing of wounds resulting from mechanical injuries, chemical injuries, burns, radiation injuries, surgical operations or pathological conditions comprising administering a therapeutically effective amount of the composition.

The present invention also relates to the composition comprising zebularine, or a pharmaceutically acceptable salt thereof, and retinoic acid, or a pharmaceutically acceptable salt thereof, and at least one pharmaceutically acceptable carrier for use in the promotion or as a medicament to promote tissue regeneration and healing of wounds resulting from mechanical injuries, chemical injuries, burns, radiation injuries, surgical operations or pathological conditions wherein the composition promotes tissue regeneration and healing of chronic wounds and non-healing wounds such as in the case of pressure ulcers, diabetic complications and peripheral artery disease.

The present invention also relates to the composition comprising zebularine, or a pharmaceutically acceptable salt thereof, and retinoic acid, or a pharmaceutically acceptable salt thereof, and at least one pharmaceutically acceptable carrier for use in the promotion or as a medicament to promote tissue regeneration and healing of wounds resulting from mechanical injuries, chemical injuries, burns, radiation injuries, surgical operations or pathological conditions wherein regeneration of complex tissues occurs which encompasses dermis and epidermis, hair, hair follicles and sweat glands, cartilage, neurons, blood vessels, muscles.

The present invention also relates to the composition comprising zebularine, or a pharmaceutically acceptable salt thereof, and retinoic acid, or a pharmaceutically acceptable salt thereof, and at least one pharmaceutically acceptable carrier for use as a medicament to promote tissue regeneration and healing of wounds resulting from mechanical injuries, chemical injuries, burns, radiation injuries, surgical operations or pathological conditions wherein the medicament is in the form of an ointment, cream, spray, injection or tablet.

The following terms used in the description and claims shall have the following meanings:
It is to be understood that the term "wound" encompasses any injury of living tissue caused by impact, pressure and other mechanical factors, heat, cold, radiation, chemical agents, ischemia, and other biological agents. In particular the term "wound" encompasses skin lesions but also lesions of other tissues and organs.

As used herein the term "wound healing" refers to a post injury repair process which involves debridement of foreign bodies and necrotic tissues within the wound area and cell proliferation leading to the restoration of tissue continuity with or without scar formation.

As used herein the term "regeneration" refers to a process of restoration of lost tissues, structures and appendages that occurs either spontaneously or under pharmacological treatment.

As used herein the term "complex tissue" refers to a tissue containing at least two different cell types which function together.

As used herein the term "chronic wound" or "non-healing wound" refers to a wound that does not fully heal in the amount of time considerably exceeding that predicted for normal healing; typically wounds that do not heal within 3 months are considered chronic. Chronic wounds or non-healing wounds are often ischemic wounds and ulcers, those of diabetic patients, but are not limited to those.

The term "CpG sites" or "CpG" refers to a DNA sequence where a cytosine nucleotide is followed by a guanine nucleotide along its 5' → 3' direction

The term "DNA methyltransferase" or "DNMT" refers to the DNMT1, DNMT3A and DNMT3B proteins. DNMT1 is a maintenance methyltransferase that is responsible for copying DNA methylation patterns to the daughter strands following DNA replication, whereas DNMT3A and DNMT3B are thought to be *de novo* methyltransferases that set up DNA methylation patterns early in development.

As used within the term "IDO" refers to three different proteins that can catabolize tryptophan: IDO-1, IDO-2 and TDO.

In one embodiment, zebularine is administered at early stage of wound healing. It is to be understood that the term "early stage" of wound healing can encompass immunological response from immediately after wounding.

### BRIEF DESCRIPTION OF THE DRAWINGS

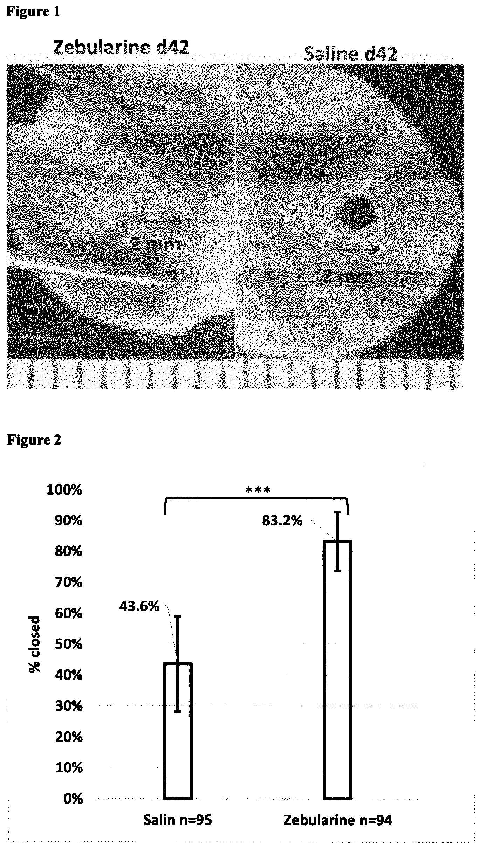

Figure 1. presents the representative photographs of ear pinnae hole closure 42 days post injury in the zebularine treated and *vehiculum* (saline) administered control mice. The ear pinnae of the mouse receiving zebularine in intraperitoneal injections was almost completely closed within approximately 30 days post injury, while in the saline administered control, the ear pinnae hole was only slightly decreased as compared to the initial excision of 2 mm diameter. Zebularine was administered 7 times (1000 mg/kg body weight): on the day of injury directly after excision and 1, 2, 3, 4, 7, and 10 days after injury.
Figure 2. presents the results of ear pinnae healing for 47 zebularine treated (n=94 ears) and 48 control mice (n=95 ears) expressed as the percentage of ear hole closure area on the day 42 after injury.

Zebularine was administered 7 times (1000 mg/kg body weight): on the day of injury directly after excision and 1, 2, 3, 4, 7, and 10 days after injury Statistical significance was determined using heteroscedastic two tailed Student's t-test. The differences with p-values lower than 0.001 are marked with triple asterisks (^{∗∗∗}).

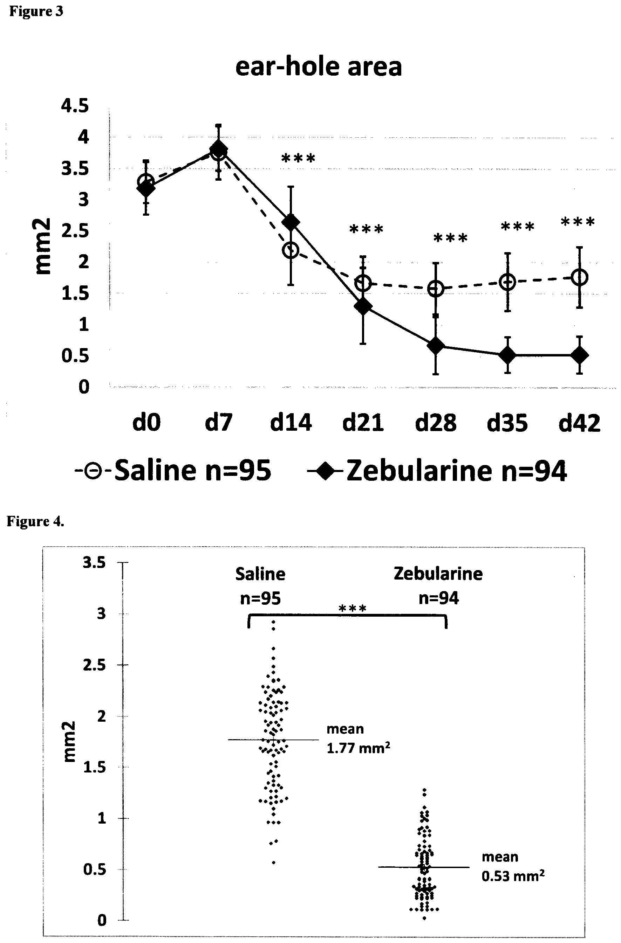

Figure 3. presents the timeline of ear pinnae hole closure determined by the ear hole area measured directly after injury and 7, 14, 21, 28, 35 and 42 days after injury for 47 zebularine treated (n=94 ears) and 48 control mice (n=95 ears).

The mean ear hole sizes in the zebularine treated and control mice remained similar during the initial several days after injury. On the 14 day post injury, the mean ear hole area was a little bigger, while from the day 21 post injury, it was recorded to be significantly smaller in the zebularine treated animals as compared to the control ones. The maximal closure was recorded from the day 35 post injury.

Zebularine was administered 7 times (1000 mg/kg body weight): on the day of injury directly after excision and 1, 2, 3, 4, 7, and 10 days after injury.

Statistical significance was determined using two-tailed Mann-Whitney test. The differences with p-values lower than 0.001 are marked with triple asterisks (^{∗∗∗}).

Figure 4. presents the distributions of ear pinnae healing results for 47 (n=94 ears) zebularine treated and 48 (n=95 ears) control mice 42 days after injury.

Each dot represents the area of a single ear pinnae hole.

Zebularine was administered 7 times (1000 mg/kg body weight): on the day of injury directly after excision and 1, 2, 3, 4, 7, and 10 days after injury.

Statistical significance was determined using two-tailed Mann-Whitney test. The differences with p-values lower than 0.001 are marked with triple asterisks (^{∗∗∗}).

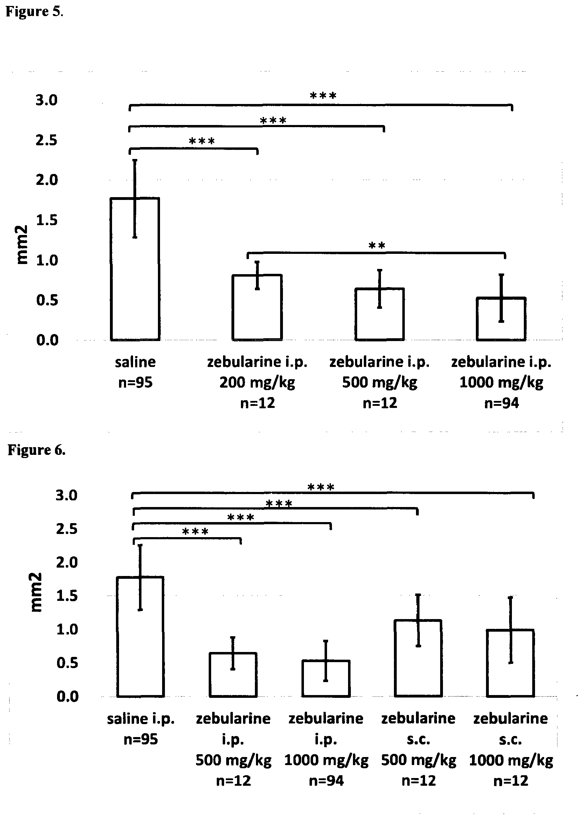

Figure 5. presents zebularine dose-effect on regenerative ear hole closure.

Mean ear pinnae hole sizes on the day 42 post injury examined for 200, 500 and 1000 mg/kg body weight.

Zebularine was administered 7 times: on the day of injury directly after excision and 1, 2, 3, 4, 7, and 10 days after injury.

Statistical significance was determined using two-tailed Mann-Whitney test. The differences with p-values lower than 0.001 are marked with triple asterisks (^{∗∗∗}).

Figure 6. presents the effect of subcutaneous and intraperitoneal zebularine delivery on regenerative ear hole closure.

Mean ear pinnae hole sizes were examined on the day 42 post injury.

Zebularine was administered 7 times (1000 mg/kg body weight): on the day of injury and 1, 2, 3, 4, 7, 10 days after injury.

Statistical significance was determined using two-tailed Mann-Whitney test. The differences with p-values lower than 0.001 are marked with triple asterisks (^{∗∗∗}).

Figure 7. presents the gene expression changes observed for selected pluripotency, developmental and cell cycle genes under zebularine treatment in the injury/regeneration area. Relative gene expression was determined using qPCR with *Tbp* and beta-actin *(Actb)* as the reference transcripts. Mean transcript levels were calculated for six animals (n=8 ears) at each time point. Statistical significance was determined using two-tailed Mann-Whitney test. The significant results (p< 0.05) are marked with an asterisk (^{∗}).

Figure 8. presents the decrease in DNA methylation expressed as 5-methyl-cytosine content in the injury/regeneration area under zebularine treatment.

Mean CpG methylation levels were determined for six animals (n=8 ears) at each time point. Statistical significance was determined using heteroscedastic two tailed Student's T-test. The result lower than 0.001 is marked with a triple asterisk (^{∗∗∗}).

Figure 9. presents the representative photographs of complete regenerative ear hole closure under synergistic action of zebularine and retinoic acid.

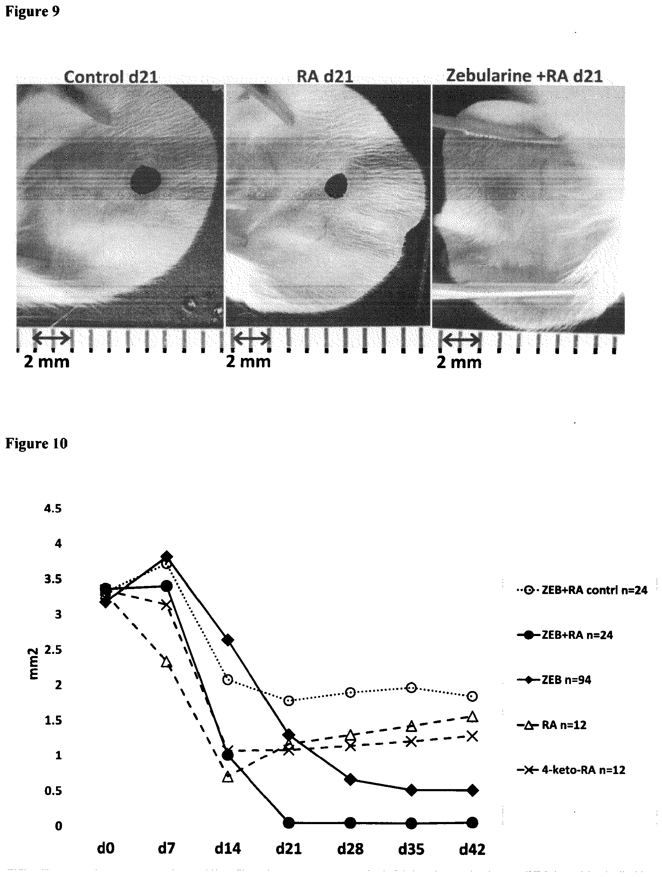

Complete ear hole closure was observed in mice receiving zebularine and retinoic acid in intraperitoneal injections within 21 days post injury. Zebularine (1000 mg/kg body weight) was administered 7 times: on the day of injury and 1, 2, 3, 4, 7, 10 days after injury. Retinoic acid, RA, (16 mg/kg body weight) was administered 6 times: on the day of injury and 2, 4, 7, 9 and 11 days after injury weight. The *vehiculum* administered controls showed no significant ear hole closure, while partial closure was observed in the mice treated with RA alone. Figure 10. presents the potentiation of zebularine pro-regenerative activity by synergistic action of retinoic acid (RA).

Timeline of ear pinnae hole closure was determined directly after injury and 7, 14, 21, 28, 3,5 and 42 days after injury for 12 mice (n= 24 ears) receiving combined zebularine and RA treatment (ZEB+RA), 6 mice (n=12 ears) receiving RA alone (RA), 6 mice (n=12 ears) receiving 4-keto-retinoic acid (4-keto-RA), and 12 mice (n=24 ears) administered with vehiculum (ZEB+RA contrl). The timelines were juxtaposed to that for zebularine delivered alone (ZEB, Figure 3). Complete ear hole closure was recorded on the day 21 after injury in the mice receiving the combined treatment with zebularine and RA

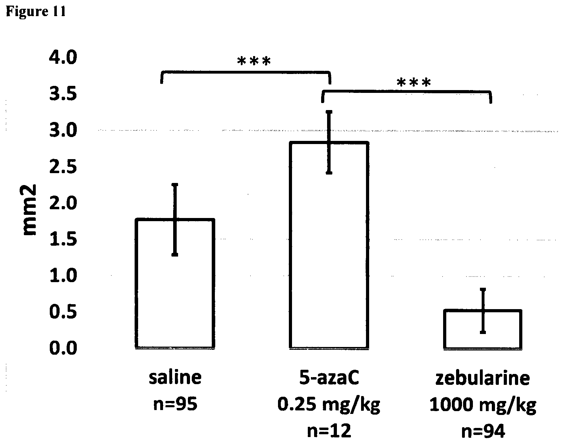

Figure 11. presents 5-azacytidine effect on ear hole closure.

5-azacytidine (5-azaC) (0.25 mg/kg body weight) was delivered intraperitoneally 7 times: on the day of injury and 1, 2, 3, 4, 7, 10 days after injury. Mean ear pinnae hole area was measured on the day 42 after injury. The results were compared to those for the mice treated with zebularine (1000 mg/kg body weight) and saline according to the same schedule. Statistical significance was determined using two-tailed Mann-Whitney's test. The differences with p-values lower than 0.001 are marked with triple asterisks (^{∗∗∗}).

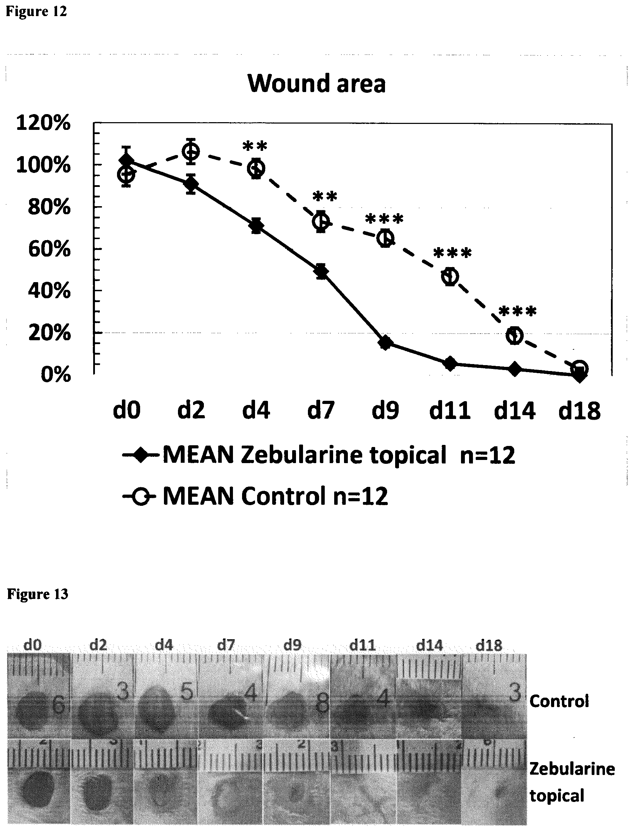

Figure 12. presents the effect of topical zebularine treatment on dorsal skin wound healing.

Wound closure was determined directly after injury and 2, 4, 7, 11, 14 and 18 days after injury for 6 zebularine treated (n=12 wounds) and 6 control mice (n=12 wounds). Error bars show SEM. Statistical significance was determined using two-tailed Mann-Whitney's test. The differences with p-values lower than 0.005 and 0.001 are marked with double (^{∗∗}) and triple asterisks (^{∗∗∗}), respectively.

Figure 13. presents the representative photographs of dorsal skin wounds in the mice receiving zebularine topically for five days following injury and the controls. The initial wound diameter was 6 mm. The wound treated with zebularine displayed complete closure 11 days after injury. The same wound of the same individual is shown throughout all time points.

### EXAMPLES

The invention is further illustrated by the following non-limiting examples.

### Example 1

### Determining the effect of zebularine on complex tissue regeneration

In order to evaluate the regenerative effect of zebularine, through-and-through holes of 2 mm diameter were made in the ear pinnae of the BALB/c 2-month-old female mice using scissor style ear punch. The mice were randomly divided into two groups consisting of six individuals each. The mice of one group were intraperitoneally injected with 7 doses of zebularine dissolved in saline (50 mg/ml), 1000 mg/kg body weight, on the day of injury directly after punching, and 1, 2, 3, 4, 7, 10 days after injury. The second, control group, was administered with saline.

The animal study protocols were approved by the Local Ethics Committee for Animal Experimentation at the University of Science and Technology in Bydgoszcz, Poland (Permit No. 50/2016).

The ears were photographed every 7 days, for 42 days, and ear hole closure was tracked by determining the area using computer-assisted image analysis. The experiment was repeated 8 times. Collectively, the experiment was performed for 47 zebularine treated and 48 saline (vehiculum) administered control mice. While minor effects of ear hole closure were recorded in the control groups, similar to those reported in the literature, extensive ear pinnae restoration was observed in the zebularine treated animals, which was manifested by broad rings of newly formed tissues surrounding the injury area as demonstrated in the representative photographs (Figure 1). The mean ear hole areas determined on the day 42 post injury were 0.53+/-0.3 and 1.77+/-0.48 sq mm for zebularine treated and saline controls, respectively. The results expressed as percentage of wound closure correspond to 83.2% and 43.6% for the zebularine treated and control group, respectively (Figure 2).The timeline of regeneration process is presented in Figure 3. Significantly better ear hole closure under zebularine treatment was recorded from the day 21 after injury, while maximal closure on the day 35. The distributions of ear hole areas on the day 42 after injury were plotted as dot diagrams, where each dot represents an individual ear (Figure 4). The diagrams not only show the biological variation in response to injury and zebularine treatment but clearly expose the contrast between the control and zebularine treated groups.

In order to evaluate dose dependent regenerative response, the effects of zebularine doses of 200, 500, and 1000 mg/kg body weight were examined (Figure 5). The effect of intraperitoneal zebularine delivery on the ear pinnae hole closure was compared with that of subcutaneous one. Subcutaneous injections gave statistically significant response but proved less effective than intraperitoneal delivery (Figure 6).

### Example 2.

### Determining the effect of systemic zebularine treatment on the expression of pluripotency and developmental genes

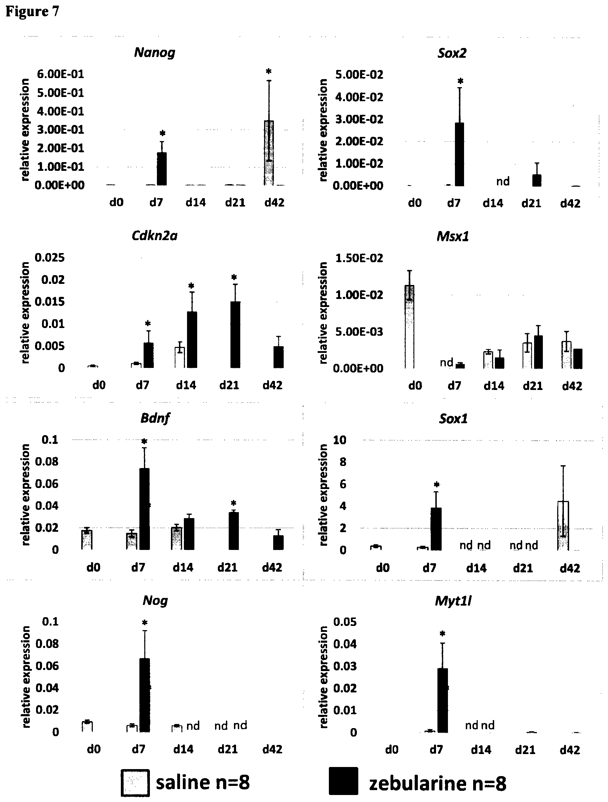

In order to determine the effect of zebularine on the expression of pluripotency, developmental and cell cycle genes, the transcript levels for a panel of selected genes including *Cdkn2a, Msx1, Nanog, Sox2, Bdnf, Sox1, Nog,* and *Myt1l* were examined in the injury/repair area, in zebularine treated and control mice at the day 7, 14, 21 and 42 following injury in addition to unwounded ear pinnae designated as day 0. For the purpose, the ear punched mice were sacrificed at selected time points, the ears were collected on RNAlater reagent (Qiagen), stored at -80 °C until the rings surrounding the holes were excised using a 3 mm biopsy punch, following RNA extraction from thus obtained tissues using RNeasy kit (Qiagen). The levels of selected transcripts were determined for 6 mice (8 ears) for each time point using real-time PCR quantitation and *Tbp* and *Actb* as the reference genes. The PCR primers are listed in Table 1. Significantly enhanced expression of the selected transcripts in the mouse treated with zebularine was observed on the day 7 after injury. The most remarkable changes were found for the pluripotency genes, *Nanog* and *Sox2* (Figure 7).

### Example 3.

### Determining the effect of systemic zebularine treatment on the global DNA methylation levels in the injury and regeneration area

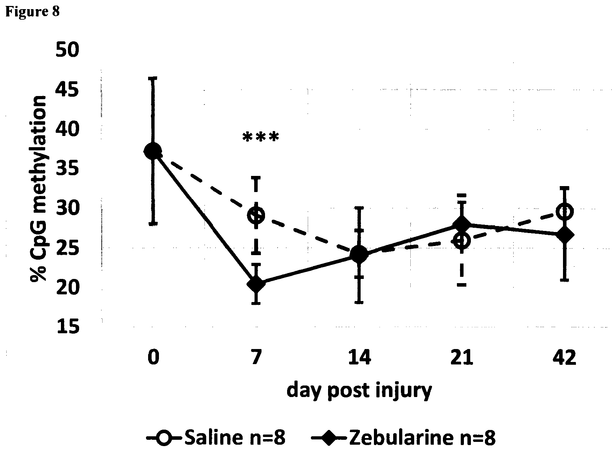

DNA methylation in promoter regions is one of key epigenetic mechanisms responsible for gene repression. In order to determine the zebularine demethylating effect on DNA in the regenerating tissues, the content of 5-methylcytosine was compared in the tissues collected from the rings surrounding the ear pinnae holes in the zebularine treated and control animals. For the purpose, the ear punched zebularine treated and control mice were sacrificed at the days 7, 14, 21, and 42 after injury. The ears were collected, and the rings surrounding the holes were excised with a 3 mm biopsy punch, following genomic DNA extraction from thus obtained tissues using DNeasy kit (Qiagen). The levels of 5-methylcytosine were estimated using 5-mC DNA ELISA Kit (ZYMO, D5325) in 100 ng aliquots of genomic DNA. The results demonstrate a significant decrease in DNA methylation on the day 7 following injury in zebularine treated animals (Figure 8), thus indicating that DNA demethylation accompanies the regenerative response in the injury area.

### Example 4. (according to the claimed invention)

### Synergistic action of zebularine and retinoic acid (RA) to stimulate regenerative responses

While retinoic acid (RA) stimulates expression of multiple genes involved in development, zebularine, as a demethylating agent is supposed to re-activate genes repressed by DNA methylation in the course of development. Therefore, the combination of these two agents was supposed to potentiate the regenerative response in the ear pinnae injury model. The effect of combined delivery of zebularine and RA on ear pinnae hole closure was compared with those of RA or zebularine delivered alone. Trough-and-through holes of 2 mm diameter were made in the ear pinnae of the BALB/c mice using scissor style ear punch. The mice were randomly divided into four groups consisting of six 2-month-old female individuals each. The mice of the first group were intraperitoneally injected with zebularine 1000 mg/kg body weight, dissolved in saline (50 mg/ml) and RA 16 mg/kg body weight suspended in 3% dimethyl sulfoxide in rapeseed oil (2 mg/ml). The injections of zebularine were made, on the day of injury directly after punching, and 1, 2, 3, 4, 7, 10 days after injury, while those of RA on the day of injury directly after punching, and 2, 4, 7, 9 and 11 days after injury. The second group was administered with RA 16 mg/kg body weight alone, the third one with 16 mg/kg body weight of 4-keto retinoic acid (4-keto RA), which is a cellular metabolite generated through cytochrome oxidation of RA, and the fourth control group received *vehiculum* only according to the same schedule. The mouse ears were photographed every 7 days, for 42 days, and ear hole closure was tracked by determining the area using computer-assisted image analysis. Partial ear hole closure was noted in the control groups administered with RA and 4-keto RA and a very limited ear hole closure was observed in the mice receiving vehiculum alone. The combined treatment with zebularine and RA resulted in accelerated and complete closure as shown in the representative photographs (Figure 9). The timeline of ear pinnae hole-closure for the animals treated with the combination of RA and zebularine contrasted with those receiving either zebularine, RA or *vehiculum* alone are presented in Figure 10.

### Example 5. (not part of the claimed invention)

### The effect of 5-azacytidine on ear hole closure

Similarly as zebularine, 5-azacytidine is a nucleoside inhibitor of DNA methyltransferases. The effect of 5-azacytidine on ear hole closure was examined using the same delivery schedule as for zebularine, but a much lower dose (0.25 mg/kg body weight) was applied. 5-azacytidine, unlike zebularine is not subjected to rapid metabolic conversion to uridine, so it is more active but also more toxic than zebularine. The mouse ears were photographed every 7 days, for 42 days, and ear hole closure was tracked by determining the area using computer-assisted image analysis. The comparison of results obtained for 6 mice that received i.p. injected 5-azacytidine with those recorded for zebularine and saline treated animals showed that 5-azacytidine did not stimulate ear hole closure, and, on the contrary, it inhibited the limited physiological closure observed in saline controls (Figure 11). The example indicates that any freely selected DNA methyltransferase inhibitor cannot replace zebularine as a pro-regenerative agent.

### Example 6

### The effect of topically delivered zebularine on skin wound healing

The effect of topically delivered zebularine on wound healing was determined using the dorsal skin excision model. Six 2-month-old female mice of the BALB/c strain were used for the experiment. Prior to the injury, the mice were anaesthetized. The dorsal skin was shaved and disinfected. Two symmetrical full-thickness excisional wounds were made in the dorsum using a biopsy punch of 6 mm diameter. Ten microliter of zebularine suspension (200 mg per 1 ml of saline) was dropped directly onto the wound immediately after wounding and once daily for the next four days. Promptly after zebularine application, the wounds were covered with a transparent dressing, and next, the dressing was fastened with adhesive plaster wrapped around the mouse. An analogical experiment was performed for six mice of control group that were not treated with zebularine. The animal study protocol was approved by the Local Ethics Committee for Animal Experimentation at the University of Science and Technology in Bydgoszcz, Poland (Permit No. 49/2016).

The wounds were photographed on the day of injury (d0) and on the day 2, 4, 7, 9, 11, 14, and 18 after wounding. The wound closure progress was tracked by determining wound area using computer-assisted image analysis. Zebularine treatment significantly accelerated wound healing. As demonstrated in Figure 12, the time of wound closure was decreased by approximately 7 days in the zebularine treated mice. Complete wound closure was observed on the day 11 for zebularine treated mice and on the day 18 for the control ones (Figure 13).

**Table 1**

| Nucleotide sequences of PCR primers used for quantitating transcript levels. | | |
|---|---|---|
| Gene | Primer forward | Primer reverse |
| *Actb* | GGCTGTATTCCCCTCCATCG | CCAGTTGGTAACAATGCCATGT |
| *Bdnf* | ATTAGCGAGTGGGTCACAGC | ATTGCGAGTTCCAGTGCCTT |
| *Cdkn2a* | TGTGTACCGCTGGGAACG | GCTCTGCTCTTGGGATTGG |
| *Msx1* | TCCCCGCTTCTCCCCGCCC | CGGTTGGTCTTGTGCTTGC |
| *Myt1l* | CGGAACCCAGACATGGAGGT | CTACAGGCAAGTCCCAGCAA |
| *Nanog* | TACCTCAGCCTCCAGCAGAT | CCAGATGCGTTCACCAGATA |
| *Nog* | CCAGCACTATCTACACATC | TCTCGTTCAGATCCTTCTCCT |
| *Sox1* | CTTCATGGTGTGGTCCCG | TTGCTGATCTCCGAGTTGTG |
| *Sox2* | GGGAGAAAGAAGAGGAGAGAGA | CGATTGTTGTGATTAGTTTTTGGA |
| *Tbp* | GAGAGCCACGGACAACTGCG | GGGAACTTCACATCACAGCTC |

### REFERENCES

Andrade, A. F., K. S. Borges, A. M. Castro-Gamero, V. S. Silveira, V. K. Suazo, J. C. Oliveira, D. A. Moreno, R. G. de Paula Queiroz, C. A. Scrideli and L. G. Tone (2014). "Zebularine induces chemosensitization to methotrexate and efficiently decreases AhR gene methylation in childhood acute lymphoblastic leukemia cells." Anticancer drugs 25(1): 72-81. Arimany-Nardi, C., E. Errasti-Murugarren, G. Minuesa, J. Martinez-Picado, V. Gorboulev, H. Koepsell and M. Pastor-Anglada (2014). "Nucleoside transporters and human organic cation transporter 1 determine the cellular handling of DNA-methyltransferase inhibitors." British journal of pharmacology 171(16): 3868-3880.
Bastakoty, D., S. Saraswati, J. Cates, E. Lee, L. B. Nanney and P. P. Young (2015). "Inhibition of Wnt/β-catenin pathway promotes regenerative repair of cutaneous and cartilage injury." The FASEB Journal 29(12): 4881-4892.
Ben-Kasus, T., Z. Ben-Zvi, V. E. Marquez, J. A. Kelley and R. Agbaria (2005). "Metabolic activation of zebularine, a novel DNA methylation inhibitor, in human bladder carcinoma cells." Biochemical pharmacology 70(1): 121-133.
Beumer, J. H., E. Joseph, M. J. Egorin, R. S. Parker, D. Z. D'argenio, J. M. Covey and J. L. Eiseman (2006). "A mass balance and disposition study of the DNA methyltransferase inhibitor zebularine (NSC 309132) and three of its metabolites in mice." Clinical cancer research 12(19): 5826-5833.
Blakytny, R. and E. Jude (2006). "The molecular biology of chronic wounds and delayed healing in diabetes." Diabet Med 23(6): 594-608.
Brem, H., T. Jacobs, L. Vileikyte, S. Weinberger, M. Gibber, K. Gill, A. Tarnovskaya, H. Entero and A. J. Boulton (2003). "Wound-healing protocols for diabetic foot and pressure ulcers." Surg Technol Int 11: 85-92.
Buckley, G., A. D. Metcalfe and M. W. Ferguson (2011). "Peripheral nerve regeneration in the MRL/MpJ ear wound model." J Anat 218(2): 163-172.
Chadwick, R. B., L. Bu, H. Yu, Y. Hu, J. E. Wergedal, S. Mohan and D. J. Baylink (2007). "Digit tip regrowth and differential gene expression in MRL/Mpj, DBA/2, and C57BL/6 mice." Wound Repair Regen 15(2): 275-284.
Cheng, J. C., C. B. Matsen, F. A. Gonzales, W. Ye, S. Greer, V. E. Marquez, P. A. Jones and E. U. Selker (2003). "Inhibition of DNA methylation and reactivation of silenced genes by zebularine." Journal of the National Cancer Institute 95(5): 399-409.
Cheng, J. C., C. B. Yoo, D. J. Weisenberger, J. Chuang, C. Wozniak, G. Liang, V. E. Marquez, S. Greer, T. F. Orntoft and T. Thykjaer (2004). "Preferential response of cancer cells to zebularine." Cancer cell 6(2): 151-158.
Clark, L. D., R. K. Clark and E. Heber-Katz (1998). "A new murine model for mammalian wound repair and regeneration." Clin Immunol Immunopathol 88(1): 35-45.
Dock, H., A. Theodorsson and E. Theodorsson (2015). "DNA methylation inhibitor zebularine confers stroke protection in ischemic rats." Translational stroke research 6(4): 296-300.
Falcone, R. E. and J. F. Nappi (1984). "Chemotherapy and wound healing." Surg Clin North Am 64(4): 779-794.
Fitzgerald, J., C. Rich, D. Burkhardt, J. Allen, A. S. Herzka and C. B. Little (2008). "Evidence for articular cartilage regeneration in MRL/MpJ mice." Osteoarthritis Cartilage 16(11): 1319-1326.
Fry, E. J., H. B. Stolp, M. A. Lane, K. M. Dziegielewska and N. R. Saunders (2003). "Regeneration of supraspinal axons after complete transection of the thoracic spinal cord in neonatal opossums (Monodelphis domestica)." J Comp Neurol 466(3): 422-444.
Frykberg, R. G. and J. Banks (2015). "Challenges in the Treatment of Chronic Wounds." Adv Wound Care (New Rochelle) 4(9): 560-582.
Gieringer, M., J. Gosepath and R. Naim (2011). "Radiotherapy and wound healing: principles, management and prospects (review)." Oncol Rep 26(2): 299-307.
Gornikiewicz, B., A. Ronowicz, M. Krzeminski and P. Sachadyn (2016). "Changes in gene methylation patterns in neonatal murine hearts: Implications for the regenerative potential." BMC Genomics 17: 231.
Gornikiewicz, B., A. Ronowicz, J. Podolak, P. Madanecki, A. Stanislawska-Sachadyn and P. Sachadyn (2013). "Epigenetic basis of regeneration: analysis of genomic DNA methylation profiles in the MRL/MpJ mouse." DNA Res 20(6): 605-621.
Herranz, M., J. Martin-Caballero, M. F. Fraga, J. Ruiz-Cabello, J. M. Flores, M. Desco, V. Marquez and M. Esteller (2006). "The novel DNA methylation inhibitor zebularine is effective against the development of murine T-cell lymphoma." Blood 107(3): 1174-1177. Lalley, A. L., N. A. Dyment, N. Kazemi, K. Kenter, C. Gooch, D. W. Rowe, D. L. Butler and J. T. Shearn (2015). "Improved biomechanical and biological outcomes in the MRL/MpJ murine strain following a full-length patellar tendon injury." J Orthop Res 33(11): 1693-1703. Lee, G., E. Wolff and J. H. Miller (2004). "Mutagenicity of the cytidine analog zebularine in Escherichia coli." DNA repair 3(2): 155-161.
Leferovich, J. M., K. Bedelbaeva, S. Samulewicz, X. M. Zhang, D. Zwas, E. B. Lankford and E. Heber-Katz (2001). "Heart regeneration in adult MRL mice." Proc Natl Acad Sci U S A 98(17): 9830-9835.
Liu, H., Z. T. Xue, H. O. Sjogren, L. G. Salford and B. Widegren (2007). "Low dose Zebularine treatment enhances immunogenicity of tumor cells." Cancer Lett 257(1): 107-115. McCaw, D. L. (1989). "The effects of cancer and cancer therapies on wound healing." Semin Vet Med Surg (Small Anim) 4(4): 281-286.
Naeem, N., K. Haneef, N. Kabir, H. Iqbal, S. Jamall and A. Salim (2013). "DNA Methylation Inhibitors, 5-azacytidine and Zebularine Potentiate the Transdifferentiation of Rat Bone Marrow Mesenchymal Stem Cells into Cardiomyocytes." Cardiovascular therapeutics 31(4): 201-209.
Nittby, H., P. Ericsson, K. Fornvik, S. Stromblad, L. Jansson, Z. Xue, G. Skagerberg, B. Widegren, H. O. Sjogren and L. G. Salford (2013). "Zebularine induces long-term survival of pancreatic islet allotransplants in streptozotocin treated diabetic rats." PLoS One 8(8): e71981.
Podolak-Popinigis, J., A. Ronowicz, M. Dmochowska, A. Jakubiak and P. Sachadyn (2016). "The methylome and transcriptome of fetal skin: implications for scarless healing." Epigenomics 8(10): 1331-1345.
Porrello, E. R., A. I. Mahmoud, E. Simpson, J. A. Hill, J. A. Richardson, E. N. Olson and H. A. Sadek (2011). "Transient regenerative potential of the neonatal mouse heart." Science 331(6020): 1078-1080.
Powell, C., A. R. Grant, E. Cornblath and D. Goldman (2013). "Analysis of DNA methylation reveals a partial reprogramming of the Muller glia genome during retina regeneration." Proc Natl Acad Sci U S A 110(49): 19814-19819.
Rybinski, B., J. Franco-Barraza and E. Cukierman (2014). "The wound healing, chronic fibrosis, and cancer progression triad." Physiol Genomics 46(7): 223-244.
Sim, C. B., M. Ziemann, A. Kaspi, K. N. Harikrishnan, J. Ooi, I. Khurana, L. Chang, J. E. Hudson, A. El-Osta and E. R. Porrello (2015). "Dynamic changes in the cardiac methylome during postnatal development." Faseb j 29(4): 1329-1343.
Tan, S. J., J. Y. Fang, Y. Wu, Z. Yang, G. Liang and B. Han (2015). "Muscle tissue engineering and regeneration through epigenetic reprogramming and scaffold manipulation." Sci Rep 5: 16333.
Tan, W., W. Zhou, H.-g. Yu, H.-S. Luo and L. Shen (2013). "The DNA methyltransferase inhibitor zebularine induces mitochondria-mediated apoptosis in gastric cancer cells in vitro and in vivo." Biochemical and biophysical research communications 430(1): 250-255. Thuret, S., M. Thallmair, L. L. Horky and F. H. Gage (2012). "Enhanced functional recovery in MRL/MpJ mice after spinal cord dorsal hemisection." PLoS One 7(2): e30904.
Ueno, M., B. L. Lyons, L. M. Burzenski, B. Gott, D. J. Shaffer, D. C. Roopenian and L. D. Shultz (2005). "Accelerated wound healing of alkali-burned corneas in MRL mice is associated with a reduced inflammatory signature." Invest Ophthalmol Vis Sci 46(11): 4097-4106.
US2009/0093528A1 (2009). METHODS FOR PROMOTING COMPOSITE TISSUE REGENERATION AND USES THEREOF
US20110076678A1 (2011). Reprogramming of somatic cells. R. B. Jaenisch, MA, US), Hanna, Yaqub (Boston, MA, US), Wernig, Marius (Woodside, CA, US), Lengner, Christopher J. (Wellesley, MA, US), Meissner, Alexander (Cambridge, MA, US), Brambrink, Oliver Tobias (Cambridge, MA, US), Welstead, Grant G. (Cambridge, MA, US), Foreman, Ruth (Somerville, MA, US). United States, Whitehead Institute for Biomedical Research (Cambridge, MA, US).
US20110206644A1 (2013). Methods for transdifferentiating cells. United States, The Board of Regents of the University of Texas System (Austin, TX, US),Strassmann, Gideon (Ramat Gan, IL).
Wang, G., S. F. Badylak, E. Heber-Katz, S. J. Braunhut and L. J. Gudas (2010). "The effects of DNA methyltransferase inhibitors and histone deacetylase inhibitors on digit regeneration in mice." Regenerative medicine 5(2): 201-220.
Xia, H., M. P. Krebs, S. Kaushal and E. W. Scott (2011). "Enhanced retinal pigment epithelium regeneration after injury in MRL/MpJ mice." Exp Eye Res 93(6): 862-872.
Yakushiji, N., M. Suzuki, A. Satoh, T. Sagai, T. Shiroishi, H. Kobayashi, H. Sasaki, H. Ide and K. Tamura (2007). "Correlation between Shh expression and DNA methylation status of the limb-specific Shh enhancer region during limb regeneration in amphibians." Dev Biol 312(1): 171-182.
Yang, P.-M., Y.-T. Lin, C.-T. Shun, S.-H. Lin, T.-T. Wei, S.-H. Chuang, M.-S. Wu and C.-C. Chen (2013). "Zebularine inhibits tumorigenesis and sternness of colorectal cancer via p53-dependent endoplasmic reticulum stress." Scientific reports 3: 3219.
Yoo, C. B., R. Valente, C. Congiatu, F. Gavazza, A. Angel, M. A. Siddiqui, P. A. Jones, C. McGuigan and V. E. Marquez (2008). "Activation of p16 gene silenced by DNA methylation in cancer cells by phosphoramidate derivatives of 2'-deoxyzebularine." Journal of medicinal chemistry 51(23): 7593-7601.
Zhang, Y., I. Strehin, K. Bedelbaeva, D. Gourevitch, L. Clark, J. Leferovich, P. B. Messersmith and E. Heber-Katz (2015). "Drug-induced regeneration in adult mice." Sci Transl Med 7(290): 290ra292.

## Claims

1. A pharmaceutical composition comprising zebularine, or a pharmaceutically acceptable salt thereof, and retinoic acid, or a pharmaceutically acceptable salt thereof, and at least one pharmaceutically acceptable carrier for use in the promotion of regeneration and healing of wounds resulting from mechanical injuries, chemical injuries, burns, radiation injuries, surgical operations or pathological conditions.

2. A pharmaceutical composition comprising zebularine, or a pharmaceutically acceptable salt thereof, and retinoic acid, or a pharmaceutically acceptable salt thereof, and at least one pharmaceutically acceptable carrier for use as a medicament to promote regeneration and healing of wounds resulting from mechanical injuries, chemical injuries, burns, radiation injuries, surgical operations or pathological conditions.

3. The composition for use according to claim 1 or 2 wherein zebularine displays DNA demethylating activity.

4. The composition for use according to claim 1 or 2 wherein zebularine activates silenced genes, preferably pluripotency genes.

5. The composition for use according to claim 1 or 2, comprising administering a therapeutically effective amount of the composition of claim 1 or 2.

6. The composition for use according to claim 5 wherein the composition promotes regeneration and healing of chronic wounds and non-healing wounds such as in the case of pressure ulcers, diabetic complications and peripheral artery disease.

7. The composition for use according to claim 5 wherein tissue regeneration of complex tissues occurs which encompasses dermis and epidermis, hair, hair follicles and sweat glands, cartilage, neurons, blood vessels, muscles.

8. The composition for use according to claim 1 or 2 wherein the composition is in the form of an ointment, cream, spray, injection or tablet.

## Patentansprüche

1. Pharmazeutische Zubereitung, die Zebularin oder ein pharmazeutisch akzeptables Salz davon und Retinsäure oder ein pharmazeutisch akzeptables Salz davon und mindestens eine pharmazeutisch akzeptable Trägersubstanz enthält, zur Verwendung für die Förderung der Regeneration und Heilung von Wunden, die aus mechanischen und chemischen Verletzungen, Verbrennungen, Strahlungsverätzungen, chirurgischen Operationen oder pathologischen Zuständen resultieren.

2. Pharmazeutische Zubereitung, die Zebularin oder ein pharmazeutisch akzeptables Salz davon und Retinsäure oder ein pharmazeutisch akzeptables Salz davon und mindestens eine pharmazeutisch akzeptable Trägersubstanz enthält, zur Verwendung als Medikament für die Förderung der Regeneration und Heilung von Wunden, die aus mechanischen und chemischen Verletzungen, Verbrennungen, Strahlungsverätzungen, chirurgischen Operationen oder pathologischen Zuständen resultieren.

3. Die Zubereitung zur Verwendung nach Anspruch 1 oder 2, wobei Zebularin als DNA-Methyltransferase-Inhibitor fungiert.

4. Eine Zubereitung zur Verwendung nach Anspruch 1 oder 2, wobei Zebularin ausgebremste Gene, vorzugsweise Pluripotenzgene, aktiviert.

5. Die Zubereitung zur Verwendung nach Anspruch 1 oder 2, umfassend die Verabreichung einer therapeutisch wirksamen Menge der Zubereitung nach Anspruch 1 oder 2.

6. Die Zubereitung zur Verwendung nach Anspruch 5, wobei die Zusammensetzung die Regeneration und Heilung von chronischen Wunden und nicht heilenden Wunden fördert, wie im Fall von Druckgeschwüren, diabetischen Komplikationen und peripheren Arterienerkrankungen.

7. Die Zubereitung zur Verwendung nach Anspruch 5, wobei komplexe Gewebearten, wie Dermis und Epidermis, Haare, Haarfollikel und Schweißdrüsen, Knorpel, Neuronen, Blutgefäße und Muskeln geheilt und regeneriert werden.

8. Die Zubereitung zur Verwendung nach Anspruch 1 oder 2, wobei die Zubereitung in Form von Salbe, Creme, Spray, Injektion oder Tablette erhältlich sein soll.

## Revendications

1. Composition pharmaceutique comprenant de la zébularine, ou un sel pharmaceutiquement acceptable de celle-ci, et de l'acide rétinoïque, ou un sel pharmaceutiquement acceptable de celui-ci, et au moins un support pharmaceutiquement acceptable pour pour utilisation dans la promotion de la régénération et de la cicatrisation des plaies résultant de blessures mécaniques, de blessures chimiques, brûlures, radiolésions, opérations chirurgicales ou conditions pathologiques.

2. Composition pharmaceutique comprenant de la zébularine, ou un sel pharmaceutiquement acceptable de celle-ci, et de l'acide rétinoïque, ou un sel pharmaceutiquement acceptable de celui-ci, et au moins un support pharmaceutiquement acceptable pour utilisation comme médicamentpour favoriser la régénération et la cicatrisation des plaies résultant de blessures mécaniques, de blessures chimiques, brûlures, radiolésions, opérations chirurgicales ou conditions pathologiques.

3. Composition pour utilisation selon la revendication 1 ou 2, dans laquelle la zébularine présente une activité de déméthylation de l'ADN.

4. Composition pour utilisation selon la revendication 1 ou 2, dans laquelle la zébularine active des gènes silencieux, de préférence des gènes de pluripotence.

5. Composition pour utilisation selon la revendication 1 ou 2, elle comprend l'administration d'une quantité thérapeutiquement efficace de la composition selon la revendication 1 ou 2.

6. Composition pour utilisation selon la revendication 5, dans laquelle la composition favorise la régénération et la cicatrisation des plaies chroniques et des plaies non cicatrisantes telles que dans le cas des escarres, des complications diabétiques et de la maladie artérielle périphérique.

7. Composition pour utilisation selon la revendication 5, dans laquelle se produit une régénération tissulaire de tissus complexes qui englobe le derme et l'épiderme, les cheveux, les follicules pileux et les glandes sudoripares, le cartilage, les neurones, les vaisseaux sanguins, les muscles.

8. Composition pour utilisation selon la revendication 1 ou 2, dans laquelle la composition est sous la forme d'une pommade, d'une crème, d'un spray, d'une injection ou d'un comprimé.
